# EUROPÄISCHE PATENTSCHRIFT

(11) **EP 0 782 982 B1**
(45) Veröffentlichungstag und Bekanntmachung des Hinweises auf die Patenterteilung: **17.05.2000**
(21) Anmeldenummer: 96810878.7
(22) Anmeldetag: 17.12.1996
(51) Int. Cl.: C07C 251/48, C07C 69/635, C07C 233/11

(54) **Verfahren zur Herstellung o-Chlor-Methyl-Phenylglyoxylsäure-Derivaten**
Process for the preparation of o-chloro-methyl-phenylglyoxylic acid derivatives
Procédé de préparation de dérivés de l'acide o-chloro-méthyl-phénylglyoxylique

(30) Priorität: 03.01.1996 CH 196; 02.05.1996 CH 111296; 26.07.1996 CH 187496
(43) Veröffentlichungstag der Anmeldung: 09.07.1997
(73) Patentinhaber: Novartis AG, 4058 Basel (CH); Novartis-Erfindungen Verwaltungsgesellschaft m.b.H., 1235 Wien (AT)
(72) Erfinder: Assercq, Jean-Marie, 1870 Monthey (CH); Schneider, Hans-Dieter, 79576 Weil am Rhein (DE); Pfiffner, Albert, 8180 Bülach (CH); Pfaff, Werner, 4334 Sisseln (CH)

(56) Entgegenhaltungen:
- EP-A- 0 178 826
- EP-A- 0 363 818
- EP-A- 0 374 811
- EP-A- 0 420 091
- EP-A- 0 585 751
- WO-A-95/18789
- WO-A-95/21153
- WO-A-95/34526
- DE-A- 4 305 502

## Beschreibung

Die Erfindung betrifft Verfahren zur Herstellung einer Verbindung der Formel I 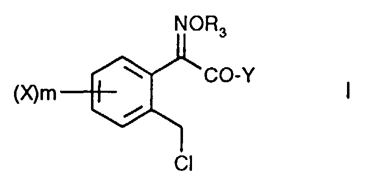 worin bedeuten:
X einen für die Reaktionen inerten Rest;
m 0 bis 4;
R₃ Wasserstoff, CH₃, CH₂F oder CHF₂;
Y eine Gruppe OR₄, N(R₅)₂, oder N(CH₃)OCH₃;
R₄ und R₅ unabhängig voneinander Wasserstoff oder C₁-C₈-Alkyl; oder
(R₅)₂ zusammen mit dem Stickstoffatom, an das sie gebunden sind, einen 5- oder 6-gliedrigen, gegebenenfalls substituierten Ring bedeuten;
dadurch gekennzeichnet, dass man umsetzt
a) eine Verbindung der Formel II 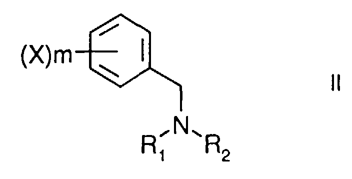 worin
   X und m die für Formel I angegebenen Bedeutungen haben und
   R₁ und R₂ unabhängig voneinander C₁-C₆-Alkyl, C₁-C₆-Alkenyl, C₁-C₆-Alkoxyalkyl, oder C₃-C₆-Cycloalkyl, oder
   R₁ und R₂ zusammen mit dem Stickstoffatom einen gegebenenfalls substituierten 6- oder 7-gliedrigen Ring, der zusätzlich zum Stickstoffatom noch ein weiteres Stickstoffatom enthalten kann, mit einer lithiumorganischen Verbindung der Formel III

      Li-R₇ (III)

      worin R₇ einen organischen anionischen Rest bedeutet, in einem aprotischen Lösungsmittel;

   b) den so erhaltenen Li-Komplex mit einer Verbindung der Formel IV

      Y₁-CO-CO-Y₁ IV

      worin
      Y₁ gleich oder verschieden eine Gruppe OR₄, N(R₆)₂, N(CH₃)OCH₃, Imidazol oder Halogen;
      R₄ C₁-C₈-Alkyl;
      R₆ C₁-C₈-Alkyl; oder
      (R₆)₂ zusammen mit dem Stickstoffatom, an das sie gebunden sind, einen 5- oder 6-gliedrigen, gegebenenfalls substituierten Ring bedeuten; und anschliessend, sofern Y₁ Imidazol oder Halogen bedeutet, dieses durch Y austauscht, worin Y die für Formel I angegebenen Bedeutungen hat;
      zu einer Verbindung der Formel V umsetzt; 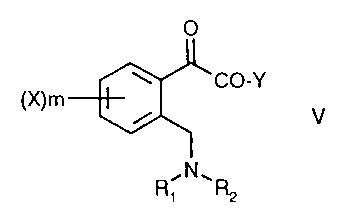
   c) diese in der Reihenfolge beliebig
      c1) mit O-Methylhydroxylamin oximiert; oder mit Hydroxylamin oximiert und anschliessend methyliert oder fluormethyliert oder difluormethyliert;
      c2) mit einem Chlorameisensäureester umsetzt.

Die Verbindungen der Formel I sind wichtige Zwischenprodukte für die Herstellung von Mikrobiziden der Methoximino-phenylglyoxylsäureester-Reihe, wie sie z.B in EP 254'426, WO 95/18789 und WO-95/21153 beschrieben werden.

Die vorstehend genannten Begriffe haben, sofern nichts anderes definiert ist, folgende Bedeutungen:

Der Rest X kann beliebig gewählt werden, sofern er gegenüber den Reaktionsbedingungen inert ist, z.B. Alkyl, Alkenyl, Phenyl, Benzyl, Nitro, Alkoxy; bevorzugt ist m = 0.

Alkylgruppen sind, je nach Anzahl der Kohlenstoffatome, geradkettig oder verzweigt und stehen beispielsweise für Methyl, Ethyl, n-Propyl, iso-Propyl, n-Butyl, sek.-Butyl, iso-Butyl, tert.-Butyl, sek. Amyl, tert. Amyl, 1-Hexyl oder 3-Hexyl.
Unter Alkenyl ist geradkettiges oder verzweigtes Alkenyl zu verstehen, wie z.B. Allyl, Methallyl, 1-Methylvinyl oder But-2-en-1-yl. Bevorzugt sind Alkenylreste mit einer Kettenlänge von 3 bis4 Kohlenstoffatomen.
Halogen bzw. Halo stehen für Fluor, Chlor, Brom oder Jod, vorzugsweise für Fluor, Chlor oder Brom.
Haloalkyl kann gleiche oder verschiedene Halogenatome enthalten, z.B. Fluormethyl, Difluormethyl, Difluorchlormethyl, Trifluormethyl, Chlormethyl, Dichlormethyl, Trichlormethyl, 2,2,2-Trifluorethyl, 2-Fluorethyl, 2-Chlorethyl, 2,2,2-Trichlorethyl, 3,3,3-Trifluorpropyl.
Alkoxy ist beispielsweise Methoxy, Ethoxy, Propyloxy, i-Propyloxy, n-Butyloxy, iso-Butyloxy, sek.-Butyloxy und tert.-Butyloxy; vorzugsweise Methoxy und Ethoxy.
Cycloalkyl ist Cyclopropyl, Cyclobutyl, Cyclopentyl oder Cyclohexyl.

Es ist aus Organic Reactions, 26, pages 1 ff (1979) bekannt, dass tert.Benzylamine mit einer lithiumorganischen Verbindung in ortho-Stellung lithiiert und diese mit einem Elektrophil in ortho-Stellung substituiert werden können. In dieser Referenz werden allerdings Oxalsäure-Derivate als Elektrophile nicht erwähnt.
Es ist ferner in EP-A-178'826, Seiten 48-75, in allgemeiner Form beschrieben, dass Phenyllithium-Verbindungen mit Oxalsäureestern zu Phenylglyoxylsäureestern reagieren; in den Beispielen werden aber keine Phenyllithium-Verbindungen hergestellt, die in ortho-Stellung durch eine Aminogruppe substituiert sind; zudem wird zur Metallierung ein Gemisch aus Butyllithium und Kalium-tert.-Butoxid verwendet.
Es wurde nun gefunden, dass Benzylamine der Formel II mit einer lithiumorganischen Verbindung und anschliessend mit einem Oxalsäure-Derivat der Formel IV zu Phenylglyoxylsäureestern der Formel V umgesetzt werden können.

Es ist zudem bekannt, dass tert.-Benzylamine mit einem Chlorameisensäureester in die entsprechenden Benzylchloride überführt werden können. So wird z.B. in Indian Journal of Chemistry, Vol.31B, S.626 (1992) ein o-Hydroxybenzyl-diethylamin mit
Chlorameisensäureethylester zum entsprechenden Benzylchlorid umgesetzt.
Es wurde ferner gefunden, dass die analoge Reaktion in guter Ausbeute auch mit solchen Benzylaminen durchgeführt werden kann, die in ortho-Stellung eine 1,2-Dioxo- oder eine 1-Ketoximino-2-oxo-Gruppe tragen, wobei diese Gruppe erhalten bleibt, was angesichts der Reaktivität dieser funktionellen Gruppe überraschend ist.

Durch das erfindungsgemässe Verfahren wird ein neuer synthetischer Zugang zu Mikrobiziden der Methoximino-phenylglyoxylsäureester-Reihe der Formel IX, wie sie z.B in EP 254'426, WO 95/18789 und WO-95/21153 beschrieben werden, eröffnet, der sich durch leichte Zugänglichkeit der Edukte, gute Ausbeuten der einzelnen Stufen und gute technische Durchführbarkeit der einzelnen Reaktionsschritte auszeichnet.
Diese neue Synthese ist in Reaktionsschema 1 dargestellt.

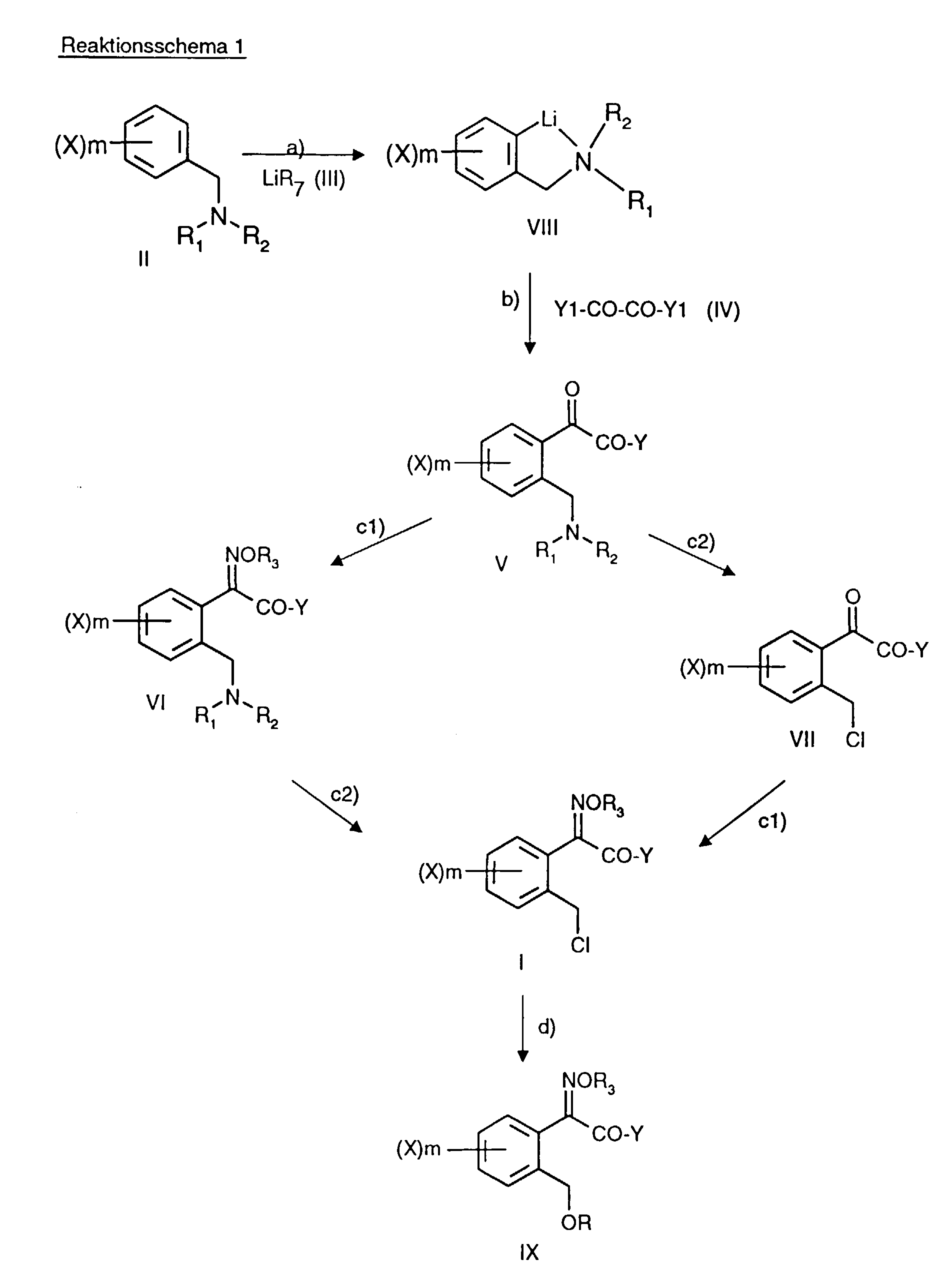

Bevorzugt werden die einzelnen Reaktionsschritte wie folgt durchgeführt:

### Reaktionsschritt a)

Reaktionstemperatur 0 bis 120°C, vorzugsweise 20°C bis zum Siedepunkt des Lösungsmittels.
Die lithiumorganischen Verbindung der Formel III ist Butyllithium, sec.Butyllithium, Hexyllithium, Lithiumdiisopropylamid (LDA), Lithiumhexamethyldisilazid oder Lithiumtetramethylpiperidid (LTMP); besonders bevorzugt ist Butyllithium.
Günstig sind 0.5-1.5 Molequivalente der lithiumorganischen Verbindung bezogen auf die Verbindung der Formel II.
Bevorzugt werden als Edukte Verbindungen der Formel II eingesetzt, worin
m 0 und
R₁ und R₂ C₁-C₆-Alkyl oder R₁ und R₂ zusammen mit dem Stickstoffatom Piperidin sind.

### Reaktionsschritt b)

Reaktionstemperatur -50°C bis zum Siedepunkt des Lösungsmittels; vorzugsweise -20 bis 30°C.
Es werden 0.9 - 4 Molequivalente des Oxalsäure-Derivates der Formel IV, bezogen auf die Verbindung der Formel II, verwendet. Das Oxalsäure-Derivat, insbesodere ein Ester, kann auch als Lösungsmittel verwendet werden.

Als Lösungsmittel eignen sich für die Reaktionschritte a) und b) ein Ether oder ein Kohlenwasserstoff oder ein Gemisch derselben;
insbesondere Hexan, Benzol, Toluol, Xylol, Tetrahydrofuran, Diethylether, Methyltert.butylether, Diisopropylether, Dimethoxyethan, Diethoxyethan, Diethoxymethan. Bevorzugt werden die beiden Reaktionsschritte im gleichen Lösungsmittelgemisch durchgeführt.

Sofern im Oxalsäure-Derivat der Formel IV Y₁ Halogen oder Imidazol bedeutet, wird das der Formel V entsprechende Glyoxylsäurehalogenid bzw. Imidazol-Derivat mit HOR₄, bzw. HN(R₅)₂ unter basischen Bedingungen zum entsprechenden Ester bzw. Amid umgesetzt. Ferner kann der Glyoxylsäureester durch Aminolyse mit HN(R₅)₂ in das gewünschte Glyoxylsäureamid überführt werden oder mit einem Alkohol umgeestert werden.

Bevorzugt wird dabei der Ethylester in den Methyl- oder n-Pentylester überführt. Als Oxalsäure-Derivat wird bevorzugt ein Ester, insbesondere der Ethylester, eingesetzt.

Nach Reaktionsschritt b) wird das Reaktionsgemisch vorteilhaft bis pH gleich oder kleiner 7 angesäuert, z.B mit einer wässrigen Säure wie Salzsäure, Schwefelsäure oder Phosphorsäure, oder mit einer wasserfreien Säure, z.B. einer Carbonsäure wie Propionsäure oder Essigsäure, oder einem Ammonium-Salz; danach wird die organische Phase mit Wasser ausgewaschen und das Produkt der Formel V durch Destillation oder Kristallisation gereinigt. Das Produkt kann auch durch saure Extraktion der Nebenprodukte gereinigt werden.
Es ist auch möglich, nach Reaktionsschritt b) ohne Reinigung des Zwischenproduktes V direkt die Reaktion c1) oder c2) anzuschliessen.

### Reaktionsschritt c1)

Die Verbindung der Formel V wird entweder mit O-Methylhydroxylamin umgesetzt oder mit Hydroxylamin bzw. einem seiner Salze, z.B. dem Hydrochlorid oder Sulfat, oximiert und anschliessend methyliert, z.B. mit Methyliodid, Methylchlorid oder Dimethylsulfat; bzw. fluormethyliert mit BrCH₂F; bzw. difluormethyliert mit CICHF₂ unter basischen Bedingungen.

### Reaktionsschritt c2)

Für den Austausch der Aminogruppe durch Chlor wird bevorzugt Chlorameisensäureethylester verwendet.
Die Reaktion kann in einem wasserfreien, aprotischen Lösungsmittel oder ohne Lösungsmittel durchgeführt werden, wobei als Lösungsmittel auch ein Chlorameisensäureester verwendet werden kann. Bevorzugte Lösungsmittel sind Kohlenwasserstoffe, Halogenkohlenwasserstoffe, Ester, Ether, Ketone, Nitrile oder ein Chlorameisensäureester, besonders bevorzugt Benzol, Toluol, Xylol, Chlorbenzol, Nitrobenzol, Petrolether, Hexan, Cyclohexan, Dichlormethan, Trichlormethan, Dichlorethan, Trichlorethan oder Chlorameisensäureethylester; insbesondere Essigsäureethylester, tert.Butylmethylether, Methylisobutylketon und Acetonitril.
Die Reaktionstemperatur ist vorzugsweise 0°C bis zum Siedepunkt des Lösungsmittels, insbesondere 20-120°C.

In gewissen Fällen ist die Durchführung der Reaktion in Gegenwart einer Base vorteilhaft, die z.B. in einer Menge von 1-50 Mol% bezogen auf die Verbindung der Formel V eingesetzt wird. Bevorzugte Basen sind Alkalimetall- oder Erdalkalimetallhydrogencarbonate oder -carbonate.
Der Chlorameisensäureester kann in beliebigem Ueberschuss eingesetzt und der nicht umgesetzte Anteil wieder zurückgewonnen werden; vorteilhaft ist die Verwendung einer Menge von 100-200 Mol% bezogen auf die Verbindung der Formel V.

Der Alkoholteil des Chlorameisensäureesters kann beliebig gewählt werden, sofern er keine unerwünschten Reaktionen eingeht; zweckmässigerweise besteht er aus höchstens 8 C-Atomen, bevorzugt sind ein gebenenfalls halogenierter-C₁-C₄₋-alkylester, ein gebenenfalls halogenierter-C₁-C₄₋-alkenylester, ein gegebenenfalls substituierter Benzyl- oder Phenylester, besonders bevorzugt ist Chlorameisensäureethylester.

R₁ und R₂ sind bevorzugt C₁-C₆-Alkyl oder
R₁ und R₂ zusammen mit dem Stickstoffatom Piperidin, Piperazin, Hexahydroazepin oder Tetrahydroisochinolin, besonders Piperidin.
Bei Verwendung eines Amines mit zwei Aminogruppen, z.B. Piperazin, können beide Aminogruppen für die Reaktion ausgenützt werden, d.h. es ist in diesem Falle nur ein halbes Molequivalent des Amines erforderlich.
Geeignete Basen sind z. B. Alkalimetall- oder Erdalkalimetall-hydroxide, -hydride, -amide, -alkanolate, -carbonate, -dialkylamide oder -alkylsilylamide, Alkylamine, Alkylendiamine, gegebenenfalls N-alkylierte, gegebenenfalls ungesättigte Cycloalkylamine, basische Heterocyclen, Ammoniumhydroxide sowie carbocyclische Amine. Beispielhaft seien Natrium-hydroxid, -hydrid, -amid, -methanolat, -carbonat, Kalium-tert.-butanolat, -carbonat, Lithiumdiisopropylamid, Kalium-bis(trimethylsilyl)-amid, Calciumhydrid, Triethylamin, Triethylendiamin, Cyclohexylamin, N-Cyclohexyl-N,N-dimethyl-amin, N,N-Diethylanilin, Pyridin, 4-(N,N-Dimethylamino)pyridin, N-Methylmorpholin, Benzyl-trimethyl-ammoniumhydroxid sowie 1,8-Diazabicyclo[5.4.0]undec-5-en (DBU) genannt.

### Reaktionsschritt d)

Die Verbindung der Formel I lässt sich mit einer Verbindung der Formel HOR, worin R einen organischen Rest bedeutet, unter basischen Bedingungen in einem Lösungsmittel nach bekannten Methoden umsetzen.

Die so erhaltene Verbindung der Formel IX kann gewünschtenfalls, sofern Y eine Gruppe OR₄ bedeutet, nach allgemein bekannten Methoden, umgeestert oder amidiert werden.

Besonders bevorzugt wird in Reaktionsschritt d) eine Verbindung der Formel I, worin m 0; R₃ Methyl und Y Methoxy oder Ethoxy bedeuten, mit einer Verbindung der Formel A1 oder A2 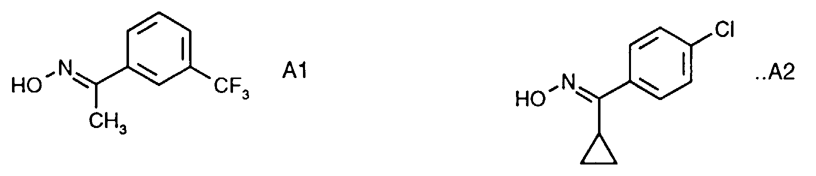 umgesetzt.
Bei der Umesterung wird vorzugsweise ein C₂-C₈-Alkylester, insbesondere der Ethylester, mit Methanol in den entsprechenden Methylester übergeführt.

Die Umsetzungen können auch unter Phasentransferkatalyse in einem organischen Lösungsmittel, z.B. Methylenchlorid oder Toluol, in Gegenwart einer wässrigen basischen Lösung, z.B. Natriumhydroxid-Lösung, sowie eines Phasentransferkatalysators, z.B. Tetrabutylammoniumhydrogensulfat, erfolgen.
Typische Reaktionsbedingungen können den Beispielen entnommen werden.

Gegenstand der Erfindung ist auch ein Verfahren zur Herstellung einer Verbindung der Formel V 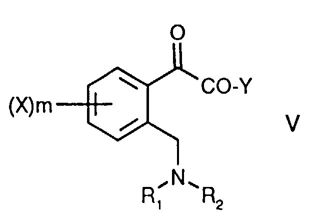 worin
X einen für die Reaktion inerten Rest;
m 0 bis 4;
R₁ und R₂ unabhängig voneinander C₁-C₆-Alkyl, C₁-C₆-Alkenyl, C₁-C₆-Alkoxyalkyl oder C₃-C₆-Cycloalkyl;
R₁ und R₂ zusammen mit dem Stickstoffatom einen gegebenenfalls substituierten 6- oder 7-gliedrigen Ring, der zusätzlich zum Stickstoffatom noch ein weiteres Stickstoffatom enthalten kann,
Y eine Gruppe OR₄, N(R₅)₂, oder N(CH₃)OCH₃;
R₄ und R₅ unabhängig voneinander Wasserstoff oder C₁-C₈-Alkyl; oder
(R₅)₂ zusammen mit dem Stickstoffatom, an das sie gebunden sind, einen 5- oder 6-gliedrigen, gegebenenfalls substituierten Ring bedeuten;
dadurch gekennzeichnet, dass man umsetzt
a) eine Verbindung der Formel II 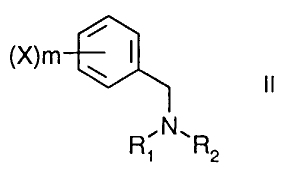 worin X, m, R₁ und R₂ die für Formel V angegebenen Bedeutungen haben; mit einer lithiumorganischen Verbindung der Formel III

   Li-R₇ (III)

   worin R₇ einen organischen anionischen Rest bedeutet, in einem aprotischen Lösungsmittel;
b) den so erhaltenen Li-Komplex mit einer Verbindung der Formel IV

   Y₁-CO-CO-Y₁ IV

   worin
   Y₁ gleich oder verschieden eine Gruppe OR₄, N(R₆)₂, N(CH₃)OCH₃, Imidazol oder Halogen;
   R₄ C₁-C₈-Alkyl;
   R₆ C₁-C₈-Alkyl; oder
   (R₆)₂ zusammen mit dem Stickstoffatom, an das sie gebunden sind, einen 5- oder 6-gliedrigen, gegebenenfalls substituierten Ring bedeuten; und anschliessend, sofern Y₁ Imidazol oder Halogen bedeutet, dieses durch Y austauscht, worin Y die für Formel I angegebenen Bedeutungen hat;
   zu einer Verbindung der Formel V umsetzt.

Ein weiterer Gegenstand der Erfindung ist ein Verfahren zur Herstellung einer Verbindung der Formel I 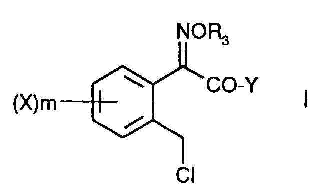 worin bedeuten:
X einen für die Reaktionen inerten Rest;
m 0 bis 4;
R₃ Wasserstoff, CH₃, CH₂F oder CHF₂;
Y eine Gruppe OR₄, N(R₅)₂, oder N(CH₃)OCH₃;
R₄ und R₅ unabhängig voneinander Wasserstoff oder C₁-C₈-Alkyl; oder
(R₅)₂ zusammen mit dem Stickstoffatom, an das sie gebunden sind, einen 5- oder 6-gliedrigen, gegebenenfalls substituierten Ring bedeuten;
dadurch gekennzeichnet, dass man eine Verbindung der Formel V 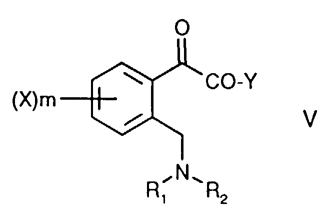 worin
X, m und Y die für Formel I angegebenen Bedeutungen haben und
R₁ und R₂ unabhängig voneinander C₁-C₆-Alkyl, C₁-C₆-Alkenyl, C₁-C₆-Alkoxyalkyl, oder C₃-C₆-Cycloalkyl oder
R₁ und R₂ zusammen mit dem Stickstoffatom einen gegebenenfalls substituierten 6- oder 7-gliedrigen Ring, der zusätzlich noch ein weiteres Stickstoffatom enthalten kann, in beliebiger Reihenfolge
   c1) mit O-Methylhydroxylamin oximiert; oder mit Hydroxylamin oximiert und anschliessend methyliert oder fluormethyliert oder difluormethyliert;
   c2) mit einem Chlorameisensäureester umsetzt.

Ein weiterer Gegenstand der Erfindung sind die neuen Verbindungen der Formel V, 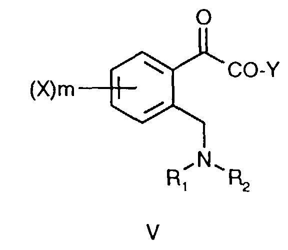 worin bedeuten:
X einen für die Reaktionen inerten Rest;
m 0 bis 4;
Y eine Gruppe OR₄, N(R₅)₂, oder N(CH₃)OCH₃;
R₄ und R₅ unabhängig voneinander Wasserstoff oder C₁-C₈-Alkyl; oder
(R₅)₂ zusammen mit dem Stickstoffatom, an das sie gebunden sind, einen 5- oder 6-gliedrigen, gegebenenfalls substituierten Ring bedeuten;
R₁ und R₂ unabhängig voneinander C₁-C₆-Alkyl, C₁-C₆-Alkenyl, C₁-C₆-Alkoxyalkyl, oder C₃-C₆- Cycloalkyl, oder
R₁ und R₂ zusammen mit dem Stickstoffatom einen gegebenenfalls substituierten 6- oder 7-gliedrigen Ring, der zusätzlich zum Stickstoffatom noch ein weiteres Stickstoffatom enthalten kann.

Bevorzugt sind solche, worin
m 0;
Y eine Gruppe OR₄;
R₄ C₁-C₈-Alkyl, insbesondere Ethyl;
R₁ und R₂ unabhängig voneinander C₁-C₆-Alkyl, insbesondere Methyl, oder
R₁ und R₂ zusammen mit dem Stickstoffatom Piperidin bedeuten.

Besonders bevorzugt ist die Verbindung der Formel Vb.

Ein weiterer Gegenstand der Erfindung ist die Verbindung der Formel VII b.

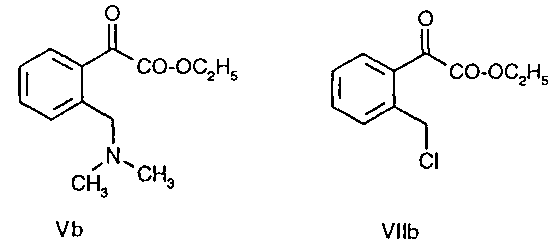

Ein weiterer Gegenstand der Erfindung ist die Verbindung der Formel Ib.

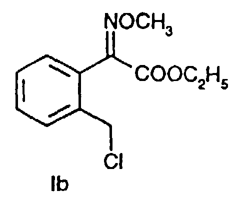

### Herstellungsbeispiele

Abkürzungen: RT = Raumtemperatur; THF = Tetrahydrofuran; h = Stunden; min = Minuten

### Beispiele 1: o-(N,N-Dimethylaminomethyl)-phenylglyoxylsäuremethylester Va

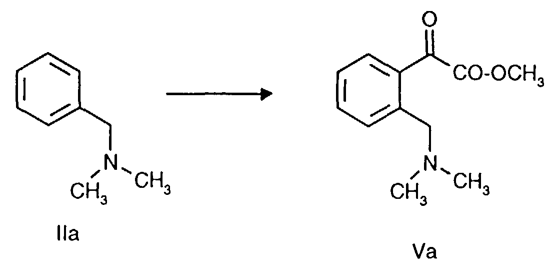

### Beisp. 1.1.

Zu einer Lösung von N-Benzyldimethylamin lla (24.1g; 0.175 mol) in Diethylether (60 ml) wird während 20 min.bei RT eine Lösung von n-Butyllithium in Hexan (15%; 107.6g; 0.25 mol) zudosiert und 3 h am Rückfluss bei ca. 50°C gehalten; danach wird diese Mischung bei - 50°C auf Dimethyloxalat (50.1g; 0.42 mol) in THF (160 ml) zudosiert; auf RT aufgewärmt, Methylchloroformat (20.3g; 0.21 mol) zugegeben, 1.5 h bei RT gerührt, im Vakuum eingedampft, der Rückstand mit je 100 ml Methylenchlorid und Wasser versetzt, die organische Phase abgetrennt und eingedampft: Der Rückstand ist 38.1 g Produkt, (Gehalt 80%; Ausbeute 79%).

### Beisp. 1.2.

Zu einer Lösung von N-Benzyldimethylamin (24,1 g; 0,175 mol) in t-Butylmethylether (60 ml) wird während 10-15 min. eine Lösung von n-Butyllithium in Toluol (20 %; 82,3 g; 0,26 mol) zudosiert. Es wird während 3 h bei 55-60°C gehalten, auf RT abgekühlt und bei -50°C zu einer Lösung von Dimethyloxalat (50,1 g; 0,42 mol) in Toluol (138,7 g) gegeben. Das Reaktionsgemisch wird auf RT erwärmt und während 13 h bei ca. 25°C gerührt, Methylchloroformiate (20,3 g; 0,21 mol) zugegeben, 1 h bei RT gerührt und im Vakuum eingedampft. Der Rückstand wird mit Methylenchlorid (100 ml) und Wasser (100 ml) versetzt, die organische Phase abgetrennt und eingedampft. Der Rückstand ist 26,6 g Produkt (Gehalt 87 %, Ausbeute 69 %).

### Beisp. 1.3.

Zu einer Lösung von N-Benzyldimethylamin (24,1 g; 0,17 mol) in Diethylether (60 ml) wird während 10-15 min. einer Lösung von n-Butyllithium in Hexan (15 %; 89,7 g; 0,21 mol) zugegeben und 3 h bei ca. 55°C am Rückfluss gehalten. Es wird auf RT abgekühlt und zu einer auf -20°C vorgekühlte Lösung von Methyloxalylchlorid (66,3 g; 0,52 mol) in Diethylether (160 ml) gegeben. Nach 30 Min rühren bei -10°C - 0°C wird das Reaktionsgemisch nochmals auf -20°C abgekühlt, mit Diethylether (100 ml) verdünnt. Unter Beibehalten der Temperatur auf -20°C bis -10°C wird eine Lösung von Natriummethylat in Methanol (30 %; 56,8 g; 0,31 mol) zugegeben. Es wird auf RT erwärmt, mit Methylenchlorid (200 ml) versetzt und über Nacht gerührt. Die Salze werden abfiltriert. Das Konzentrat wird in Toluol (200 ml) aufgenommen, die zurückbleibenden Salze werden abfiltriert und mit Toluol (50 ml) gewaschen. Das Filtrat ergibt 23,1 g Produkt (Gehalt 72 %; Ausbeute 43,1 %).

### Beisp. 1.4.

Zu einer Lösung von N-Benzyldimethylamin (13,8 g; 0,10 Mol) in Diethylether (60 ml) wird während 10-15 Min eine Lösung von n-Butyllithium in Hexan (15 %; 52 g; 0,12 mol) zusgegeben. Die erhaltene Mischung wird während ca. 3 h bei 40-45°C am Rückfluss gehalten und dann auf RT gekühlt. Danach wird diese Mischung bei -20°C bis -10°C auf eine vorgängig hergestellte Mischung aus 31 g Triethylamin (0,30 Mol), 37,9 g Methyloxalylchlorid (0,30 mol) in 160 ml Diethylether gegeben. Die erhaltene Mischung wird auf -20°C gekühlt und 32 g Methanol zugegeben, während die Temperatur auf RT ansteigt. Es wird über Nacht bei RT gerührt, abfiltriert, 2 mal mit 100 ml Diethylether gewaschen und im Vakuum eingedampft. Der Rückstand wird in 100 ml Methylenchlorid und 50 ml Wasser gelöst, die organische Phase abgetrennt und eingedampft: 16,4 g Produkt (Gehalt 69 %, Ausbeute 51 %).

### Beispiele 2: o-(N,N-Dimethylaminomethyl)-phenylglyoxylsäureethylester Vb

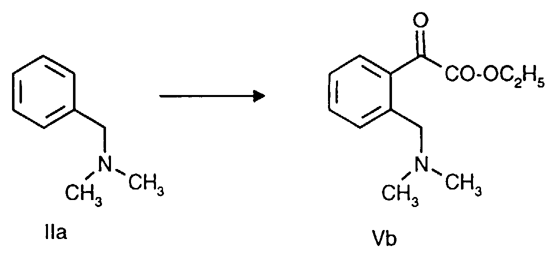

### Beisp. 2.1.

Zu einer Lösung von 48,3 g N-Benzyldimethylamin (0,35 Mol) in 120 ml Methyl-tert.-butylether wird während 15 min eine Lösung von n-Butyllithium in Hexan (15 %; 183,8 g; 0,43 Mol) zugegeben. Es wird während 4h auf 50-55°C erhitzt und danach während 30 min.zu einer kalten (-20°C) Suspension von 124 g Diethyloxalat (0,84 Mol) in 320 ml Methyl-tert.-butylether zudosiert. Danach wird auf RT erwärmt und die Reaktionsmischung mit Essigsäure (100 %; 25,2 g; 0,42 Mol), dann mit einer Mischung aus 100 g zerstossenem Eis und 200 g Wasser versetzt. Die Phasen werden getrennt, die organische Phase mit 100 ml Wasser gewaschen und im Vacuum eingedampft: 78 g (Gehalt 86,4 %; Ausbeute 82 %).

### Beisp. 2.2.

Es wird wie im vorhergehenden Beispiel verfahren; aber anstelle von Butyllithium in Hexan wird Butyllithium in Toluol (20%) verwendet. Ausbeute: 72 g (Gehalt 84,8 %; Ausbeute 74%).

### Beisp. 2.3.

Zu einer Lösung von 13,8 g N-Benzyldimethylamin (0,10 Mol) in 60 ml Diethylether wird während 10-15 Min eine Lösung von n-Butyllithium in Hexan (15 %; 54,2 g; 0,13 Mol) zugegeben und bei 35-45°C während 3 h am Rückfluss gehalten. Es wird auf RT gekühlt und während 5 Min. zu einer vorgekühlten (-20°C) Lösung von 42,2 g Ethyloxalylchlorid (0,30 Mol) in 160 ml Diethylether zudosiert. Während 30 Min wird bei 30°C gerührt und anschliessend abgekühlt auf -20°C. Bei -20°C bis 0°C werden 46 g Ethanol (1,0 mol) und 36,4 g Triethylamin (0,35 Mol) nacheinander zugegeben. Anschliessend wird auf RT erwärmt und während 1 h gerührt, die Salze abfiltriert und mit 3 x 50 ml Diethylether gewaschen. Die kombinierten Filtrate werden unter Vakuum eingedampf. Der Rückstand wird in 200 ml Methylenchlorid und 50 ml Wasser gelöst, die organische Phase abgetrennt und eingedampft:19,3 g (Gehalt 77 %; Ausbeute 63 %).

### Beispiele 3: o-(N,N-Dimethylaminomethyl)-phenylglyoxylsäure-n-pentylester Vc

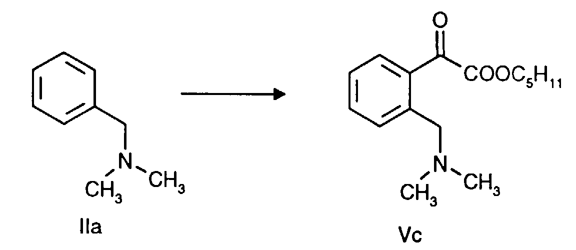

### Beisp. 3.1.

Es wird wie im Beispiel 1.1 verfahren; aber anstelle von Dimethyloxalat wird Di-n-pentyloxalat verwendet. Ausbeute: 62 %.

### Beisp. 3.2.

Zu einer Lösung von 24,1 g N-Benzyldimethylamin (0,175 Mol) in 60 ml Diethylether wird während 10-15 Min eine Lösung von n-Butyllithium in Hexan (15 %; 92.7 g; 0.22 Mol) zugegeben und bei 50-55°C während ca. 3 h am Rückfluss gehalten. Diese Mischung wird auf RT gekühlt und während 5 Min. auf eine vorgekühlte (-20°C) Lösung von 93,8 g n-Pentyloxalylchlorid (0,52 Mol) in 160 ml Diethylether zugegeben und während 30 Min gerührt; dabei lässt man die Temperatur auf 30°C ansteigen. Bei -20°C bis 0°C wird eine Mischung aus 10 g Methanol (0,31 Mol) und 32,5 g Triethylamin (0,31 Mol) zugegeben anschliessend bei RT über Nacht gerührt, unter Vakuum eingedampft und in 200 ml Methylenchlorid und 150 ml Wasser aufgenommen. Die organische Phase wird abgetrennt und eingedampft: 98,5 g (Gehalt 32 %; Ausbeute 65 %).

### Beispiel 4: o-Chlormethyl-phenylglyolylsäuremethylester Vlla

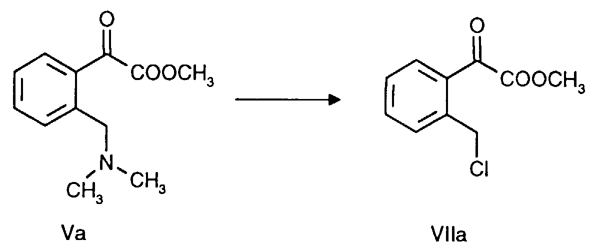

Zu einer Lösung von 18,3 g o-(N,N-Dimethylaminomethyl)-phenylglyoxylsäuremethylester Va (Gehalt 88,6 %; 73,3 Mol) in 100 ml Toluol wird bei 20-25°C 15,9 g Methylchloroformiat (165 mMol) zugegeben. Es wird bei RT über Nacht gerührt, während 1 h auf 60°C, erhitzt, abgekühlt und im Vakuum eingedampft. Man erhält 15,3 g (Gehalt 83 %; Ausbeute 82 %) des Produkts.

### Beispiel 5: o-Chlormethyl-phenylglyolylsäureethylester Vllb

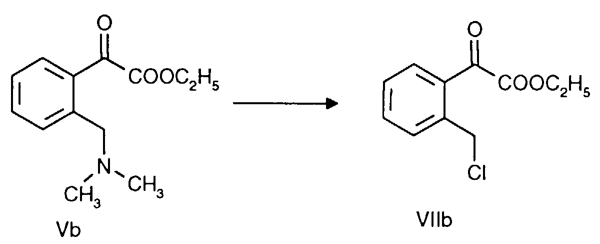

Zu einer Lösung von 10,3 g (N,N-Dimethylaminomethyl)-phenylglyoxylsäureethylester Vb (Gehalt 91,3 %; 40 mMol) in 40 ml Toluol wird bei 20-25°C 11,5 g Methylchloroformiat (119 mMol) zugegeben. Es wird bei RT über Nacht gerührt. Die Reaktionsmischung wird unter Vakuum eingedampft und ergibt 10,1 g (Gehalt 85 %; Ausbeute 94 %) des Produkts.

### Beispiel 6: o-(N,N-Dimethylaminomethyl)-phenylglyoxylsäuremethylester-O-methyloxim Via

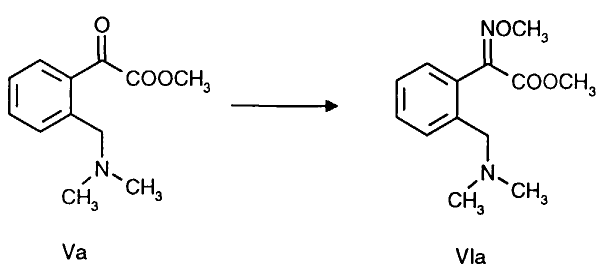

Zu einer Mischung von 4,2 g O-Methylhydroxylaminhydrochlorid (49,3 mMol), 100 g Toluol; 20 ml Methanol und 0,4 g p-Toluolsulfonsäure werden 14,4 g Ketoester Va (Gehalt 72 %; 46,9 mMol) zugegeben. Es wird während 10 h auf 50-55°C erhitzt, anschliessend am Vakuum eingedampft. Die erhaltenen Salze werden in 100 ml Methylenchlorid und 8 g Natriumcarbonat gelöst, die Salze abfiltriert und die Lösung unter Vakuum eingedampft. Man erhält 11,9 g (Gehalt 82 %, Ausbeute 83 %) des Produkts.

### Beispiel 7: o-Chlormethyl-phenylglyoxylsäure-n-pentylester-O-methyloxim lc

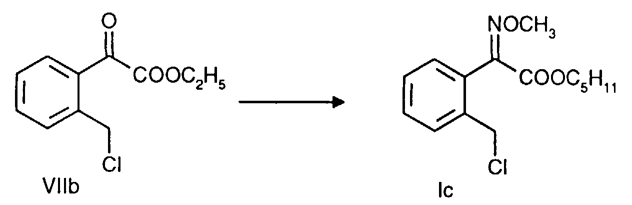

Eine Lösung von 10,1 g Ketoester Vllb (ca. 80 %; 36 mMol) und p-Toluolsulfonsäure monohydrat, (0,18 g; 1 mMol) in 39 g n-Pentanol wird während 4 h auf 90-95°C erhitzt. Anschliessend werden ca 6 g Lösungsmittel abdestilliert, mit Pentanol ersetzt und die Reaktion vervollständigt. Nach dem Abkühlten auf RT werden 3,7 g Methoxylaminhydrochlorid (44,5 mMol) zugegeben und während 20 h bei 60°C gerührt, auf RT abgekühlt und auf eine Mischung aus 60 g Eis und 40 g Wasser gegeben.. Die erhaltene Mischung wird mit wässrigem NaHCO₃ neutralisiert, die organische Phase abgetrennt, mit 30 ml Wasser gewaschen und im Vakuum eingedampft. Man erhält 10,3 g Rohprodukt als Mischung des Pentylesters lc (ca. 50 %) und des Ethylesters la (ca. 30 %).

### Beispiel 8: o-Piperidinomethyl-phenylglyozylsäureethylester Ve

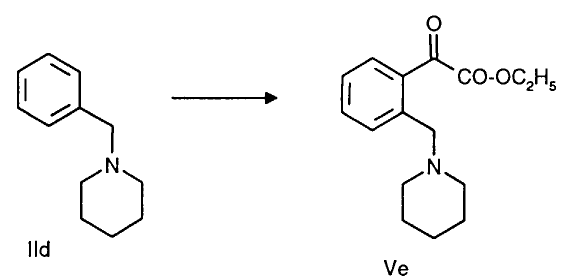

Zu einer Lösung von 31 g N-Benzylpiperidin (0,175 Mol) in 60 ml t-Butylmethylether wird während 10-15 Min eine Lösung von n-Butyllithium in Toluol (20 %; 65,6 g; 0,20 Mol) zugegeben. Es wird während ca. 18 h auf 55-60°C erhitzt, dann bei RT zu einer kalten (-20°C) Lösung von 50,1 g Diethyloxalat (0,42 Mol) in 160 ml Toluol zudosiert. Man erwärmt auf RT, rührt während 30 Min bei 20-25°C. Danach wird die Reaktionsmischung mit Essigsäure (100 %; 12,6 g; 0,21 Mol) und mit einer Mischung aus 50 g zerstossenem Eis und 100 g Wasser versetzt. Die Phasen werden getrennt, die organische Phase mit 50 ml Wasser gewaschen und unter Vacuum eingedampft: 43,2 g (Gehalt 89 %; Ausbeute 80 %).

### Beispiel 9: o-Piperidinomethyl-phenylglyolylsäure-n-pentylester Vf

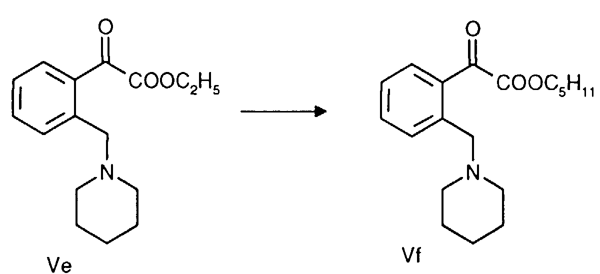

In einer Lösung von Ve (51,8 g; a 92 %; 0,2 Mol) und Natriummethoxid (95 %; 0,57 g; 10 mMol) in 180 g n-Pentanol wird unter Vakuum (200 mbar) am Rückfluss (70-75°C) laufend Ethanol abdestilliert (Rücklauf-Verhältnis 1:20). Nach ca. 2 h, wird auf RT abgekühlt und auf eine Mischung aus 50 g Eis, 50 g Wasser und 0,6 g Essigsäure gegeben. Die Phasen werden getrennt, die organische Phase mit 50 ml Wasser gewaschen und unter Vacuum eingedampft: 59,4 g (Gehalt 86,5 %; Ausbeute 98 %).

### Beispiel 10: 2-(α-Chlormethylphenyl)-2-methoximino-essigsäuremethylester la

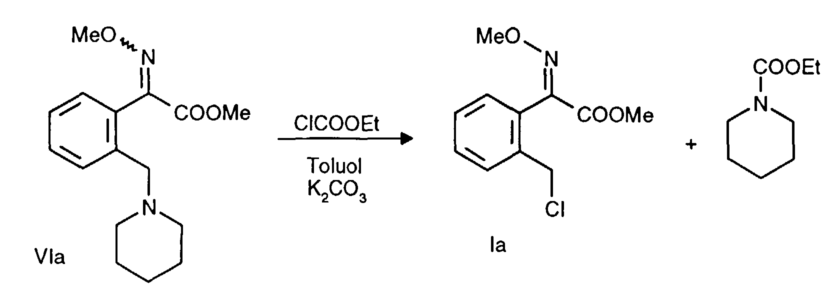

In einem 100 ml-Sulfierkolben werden 15.7 g Methoximinocarbonsäureester VIa (dest., 91.3%;
49.4 mMol) in 20 ml Toluol gelöst und mit 0.3 g (2.15 mMol) pulv. Pottasche versetzt. Anschliessend werden bei RT 7.1 ml Chlorameisensäureethylester (74.6 mMol) rasch zugetropft, wobei die RT innert 10 Min. auf 41°C ansteigt. Nach dem Abklingen der Exothermie wird auf 95°C erhitzt und mittels GC der Umsatz bestimmt: 86%. Dann werden nochmals 1.41 ml Chlorameisensäureethylester (14.8 mMol) zugegeben und nach 1/4 h erneut der Umsatz bestimmt: 98%. Nach 1 h Gesamtreaktionszeit wird das Reaktionsgemisch abgekühlt, auf Solelösung gegossen und mit 1N Salzsäure schwach angesäuert. Dann wird mit Essigester erschöpfend extrahiert und wie üblich aufgearbeitet.
Rohausbeute: 22.4 g oranges Öl.
Zweck genauer Ausbeutebestimmung der [E/Z]-Isomeren wird an Kieselgel mit EE/Hexan 1:6 chromatographiert und am Hochvakuum unter gelindem Erwärmen das mitgeschleppte Carbamat (7.18 g) abdestilliert.
Ausbeute: 11.81 g, viskoses gelbes Öl, od. 99% d.Th; Reinheit: 96.5%;Totalausbeute: 95.4% d.Th; [E/Z]-Verhältnis (GC): = 80:20.
Bezogen auf das Edukt werden in diesem Beispiel 4 Mol% Pottasche und 180 Mol% Chlorameisensäureethylester eingesetzt.

### Isomerisierung:

Beim Stehen über Nacht kristallisiert die [E]-Form im Öl aus und kann abfiltriert und mit Methylcyclohexan-tert.Butyl-methyläther gewaschen und anschliessend bis zur Gewichtskonstanz am Hochvakuum getrocknet werden.

### 1. Kristallisat: 6.37 g weisse Kristalle.

5.44 g [E/Z]-Gemisch aus der Mutterlauge werden in 20 ml Methylcyclohexan heiss gelöst, auf Raumtemperatur abgekühlt und während 5 h ein schwacher HCI-Gasstrom eingeleitet. Die zu Beginn dunkelviolette Lösung färbt sich dunkelgrün, das [E]-lsomere fällt aus und kann abfiltriert werden.

### 2. Kristallisat: 3.26 g dunkelgrüne Kristalle

Totalausbeute [E]-Isomer: 9.63 g od. 81% d.Th.

### Beispiel 11: 2-(α-Chlormethylphenyl)-2-methoximino-essigsäureethylester Ib

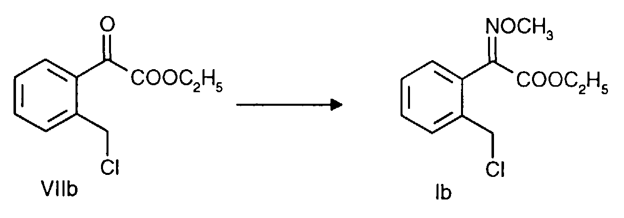

In einem Sulfierkolben werden 20 g Chlormethylketoethylester (0.071mol) zusammen mit 6.6 g o-Methylhydroxylamin * HCl(0.08 mol) und 30 g Ethanol abs. vorgelegt und auf 50-55°c geheizt. Nach 3h rühren bei 50-55°c werden anschliessend 10 g HCl-Gas (0.27 mol) innert 30 Minuten bei gleicher Temperatur eingeleitet. Nach einer Ausrührzeit von 17h bei 50-55°C wird das Reaktionsgemisch auf 0-5°C abgekühlt und mit Natronlauge auf pH 7-9 gestellt. Das ausgefallene Produkt wird abfiltriert und 3x mit je 10ml kaltem Wasser gewaschen. Anschliessend wird das wasserfeuchte Rohprodukt im Trockenschrank unter Vakuum bei 30°C getrocknet. Man erhält das Produkt (2-Chlormethyl-phenyl)methoxyimino-essigsäure-ethylester in einer Ausbeute von 87% d. Th. und einem Gehalt von 90.5% (bestehend aus: 82.8% E-lsomer und 7.7% Z-lsomer). Durch Umkristallisation kann der Gehalt an E-lsomerem auf über 95% erhöht werden. Der Schmelzpunkt des (99%) E-lsomeren beträgt 73°C. Das Z-lsomere ist bei Raumtemperatur flüssig.

### Beispiel 12: [ausserhalb der Erfindung]

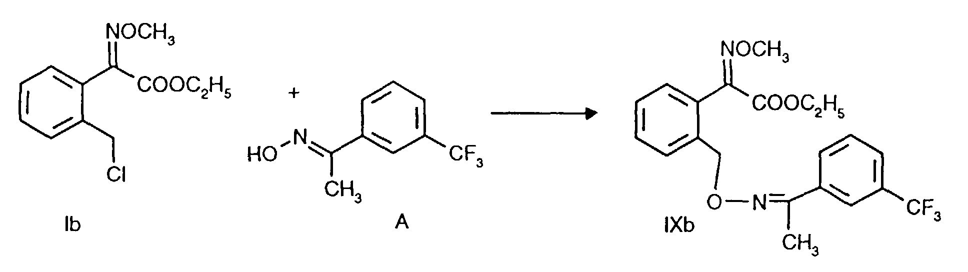

Zu einer Lösung von 7,0 g 3-Trifluormethylacetophenonoxim (A) (0,034 Mol) in 8 ml Dimethylacetamid werden in 10 Minuten 6,1 g einer 30%-igen Natriummethylat-Lösung in Methanol getropft. Bei 55 - 70°C und 250 - 50 mbar werden 3,5 ml Lösungsmittel abdestilliert. Nach Zugabe von 0,07 g Kaliumiodid werden während 20 Minuten bei 55 - 65°C 9,25 g 2-(α-Chlormethyl-phenyl)-2-methoximino-essigsäureethylester lb 90% (0,032 Mol), gelöst in 12 ml Dimethylacetamid, zudosiert. Nach einer Ausrührzeit von 3 Stunden wird das Reaktionsgemisch während 30 Minuten auf ein Gemisch von 30 ml Wasser und 18 ml Toluol bei 20 - 25°C dosiert, wobei der pH Wert mit Salzsäure 32% auf 4-5 eingestellt wird. Die untere wässrige Phase wird 2 Mal mit je 10 ml Toluol extrahiert. Die vereinigten organischen Phasen werden mit je 10 ml Wasser extrahiert. Das Lösungsmittel wird bei 60°C unter Vakuum abdestilliert. Man erhält 14,8 g Rohöl von der Verbindung lXb mit einem Gehalt von 78%. Nach Reinigung erhält man eine feste Substanz mit einem Schmelzpunkt von 47°C, Gehalt 95%.
Diese Reaktion kann auch z. Bsp in DMF oder N-Methylpyrrolidon unter den gleichen Bedingungen oder in Acetonitril mit Kaliumcarbonat als Base durchgeführt werden.

### Beispiel 13: Umesterung [ausserhalb der Erfindung]

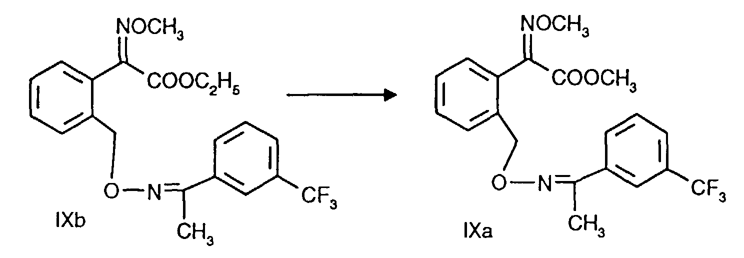

Eine Lösung von 14,8 g der Ethylester-Verbindung lXb (Gehalt: 78%; 0,027 Mol) in 53 ml Methanol und 1,5 g Natriummethylat 30% in Methanol wird für 2 Stunden bei 40 -45°C gerührt. Das Reaktionsgemisch wird bei 20 - 25°C auf ein Gemisch von 53 ml Toluol, 10 ml Wasser und 1g Salzsäure 32% dosiert, wobei der pH-Wert mit Salzsäure bei 3 - 3,5 gehalten wird. Nach Phasentrennung wird die wässrige Phase 2 Mal mit je 10 ml Toluol extrahiert. Die vereinigten organischen Phasen werden 2 Mal mit je 16 ml Wasser extrahiert. Nach Abdampfen des organischen Lösungsmittels unter Vakuum bei 60 -65°C erhält man 13,4 g Rohprodukt, das bei 55-60°C in 27 ml Methylcyclohexan gelöst wird. Während des Abkühlens auf 0-5°C fällt das Produkt aus, wird abfiltriert und mit Methylcyclohexan bei 0-5°C gewaschen. Nach Trocknen im Vakuum bei 40°C erhält man 9 g des Produkts IXa mit einem Schmelzpunkt von 69-71 °C.

## Patentansprüche

1. Verfahren zur Herstellung einer Verbindung der Formel I 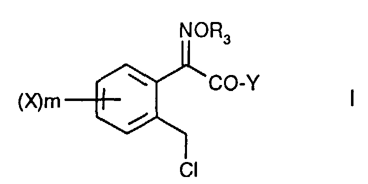 worin bedeuten:
X einen für die Reaktionen inerten Rest;
m 0 bis 4;
R₃ Wasserstoff, CH₃, CH₂F oder CHF₂;
Y eine Gruppe OR₄, N(R₅)₂, oder N(CH₃)OCH₃;
R₄ und R₅ unabhängig voneinander Wasserstoff oder C₁-C₈-Alkyl; oder
(R₅)₂ zusammen mit dem Stickstoffatom, an das sie gebunden sind, einen 5- oder 6-gliedrigen, gegebenenfalls substituierten Ring bedeuten;
dadurch gekennzeichnet, dass man umsetzt
a) eine Verbindung der Formel II 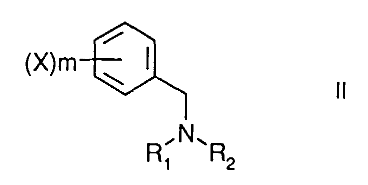 worin
X und m die für Formel l angegebenen Bedeutungen haben und
R₁ und R₂ unabhängig voneinander C₁-C₆-Alkyl, C₁-C₆-Alkenyl, C₁-C₆-Alkoxyalkyl, oder C₃-C₆-Cycloalkyl, oder
R₁ und R₂ zusammen mit dem Stickstoffatom einen gegebenenfalls substituierten 6- oder 7-gliedrigen Ring, der zusätzlich zum Stickstoffatom noch ein weiteres Stickstoffatom enthalten kann, mit einer lithiumorganischen Verbindung der Formel lll
Li-R₇ (III)
worin R₇ einen organischen anionischen Rest bedeutet, in einem aprotischen Lösungsmittel;
b) den so erhaltenen Li-Komplex mit einer Verbindung der Formel IV
Y₁-CO-CO-Y₁ IV
worin
Y₁ gleich oder verschieden eine Gruppe OR₄, N(R₆)₂, N(CH₃)OCH₃, Imidazol oder Halogen;
R₄ C₁-C₈-Alkyl;
R₆ C₁-C₈-Alkyl; oder
(R₆)₂ zusammen mit dem Stickstoffatom, an das sie gebunden sind, einen 5- oder 6-gliedrigen, gegebenenfalls substituierten Ring bedeuten; und anschliessend, sofern Y₁ Imidazol oder Halogen bedeutet, dieses durch Y austauscht, worin Y die für Formel l angegebenen Bedeutungen hat;
zu einer Verbindung der Formel V umsetzt; 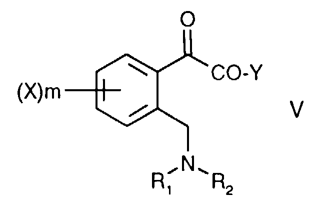
c) diese in der Reihenfolge beliebig
c1) mit O-Methylhydroxylamin oximiert; oder mit Hydroxylamin oximiert und anschliessend methyliert oder fluormethyliert oder difluormethyliert;
c2) mit einem Chlorameisensäureester umsetzt.

2. Verfahren gemäss Anspruch 1, worin der Reaktionsschritt a) bei 0°C bis 120°C und der Reaktionsschritt b) bei -50 bis +30°C durchgeführt wird.

3. Verfahren gemäss Anspruch 1, worin das Lösungsmittel für die Reaktionschritte a) und b) ein Ether oder ein Kohlenwasserstoff oder ein Gemisch derselben ist.

4. Verfahren gemäss Anspruch 3, worin der Kohlenwasserstoff Hexan, Benzol, Toluol, Xylol, und worin der Ether Tetrahydrofuran, Diethylether, Methyl-tert.butylether, Diisopropylether, Dimethoxyethan, Diethoxyethan oder Diethoxymethan ist.

5. Verfahren gemäss Anspruch 1, worin die lithiumorganischen Verbindung der Formel lll. Butyllithium, sec.Butyllithium, Hexyllithium, Lithiumdiisopropylamid (LDA), Lithiumhexamethyldisilazid oder Lithiumtetramethylpiperidid (LTMP) ist.

6. Verfahren gemäss Anspruch 5, worin die lithiumorganischen Verbindung der Formel III. Butyllithium ist.

7. Verfahren gemäss Anspruch 1, worin in der Verbindung der Formel IV Y₁ OR₃ ; insbesondere OC₂H₅ bedeutet.

8. Verfahren gemäss Anspruch 1, worin in der Verbindung der Formel II
m 0 und
R₁ und R₂ unabhängig voneinander C₁-C₆-Alkyl oder
R₁ und R₂ zusammen mit dem Stickstoffatom Piperidin sind.

9. Verfahren gemäss Anspruch 1, worin im Reaktionsschritt a) 0.5-1.5 Molequivalente der lithiumorganischen Verbindung der Formel lll bezogen auf die Verbindung der Formel II verwendet werden.

10. Verfahren gemäss Anspruch 1 worin im Reaktionsschritt b) 0.9-4 Molequivalente des Oxalsäure-Derivates der Formel IV bezogen auf die Verbindung der Formel II verwendet werden.

11. Verfahren gemäss Anspruch 1, dadurch gekennzeichnet, dass das Reaktionsgemisch nach Reaktionsschritt b) bis pH gleich oder kleiner 7 gestellt wird.

12. Verfahren gemäss Anspruch 1, worin im Reaktionsschritt c2) Chlorameisensäureethylester verwendet wird.

13. Verfahren gemäss Anspruch 1, worin in den Formel I, II und V m = 0 ist.

14. Verfahren zur Herstellung einer Verbindung der Formel V 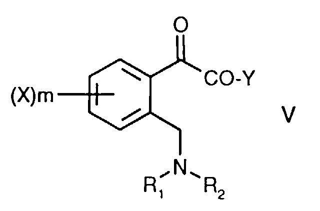 worin bedeuten:
X einen für die Reaktionen inerten Rest;
m 0 bis 4;
R₁ und R₂ unabhängig voneinander C₁-C₆-Alkyl, C₁-C₆-Alkenyl, C₁-C₆-Alkoxyalkyl oder C₃-C₆-Cycloalkyl,
R₁ und R₂ zusammen mit dem Stickstoffatom einen gegebenenfalls substituierten 6- oder 7-gliedrigen Ring, der zusätzlich zum Stickstoffatom noch ein weiteres Stickstoffatom enthalten kann,
Y eine Gruppe OR₄, N(R₅)₂, oder N(CH₃)OCH₃;
R₄ und R₅ unabhängig voneinander Wasserstoff oder C₁-C₈-Alkyl; oder
(R₅)₂ zusammen mit dem Stickstoffatom, an das sie gebunden sind, einen 5- oder 6-gliedrigen, gegebenenfalls substituierten Ring bedeuten;
dadurch gekennzeichnet, dass man umsetzt
a) eine Verbindung der Formel II 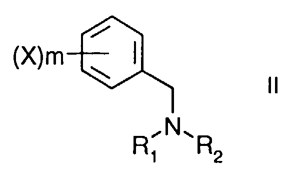 worin X, m, R₁ und R₂ die für Formel V angegebenen Bedeutungen haben; mit einer lithiumorganischen Verbindung der Formel lll
Li-R₇ (III)
worin R₇ einen organischen anionischen Rest bedeutet, in einem aprotischen Lösungsmittel;
b) den so erhaltenen Li-Komplex mit einer Verbindung der Formel IV
Y₁-CO-CO-Y₁ IV
worin
Y₁ gleich oder verschieden eine Gruppe OR₄, N(R₆)₂, N(CH₃)OCH₃, Imidazol oder Halogen;
R₄ C₁-C₈-Alkyl;
R₆ C₁-C₈-Alkyl; oder
(R₆)₂ zusammen mit dem Stickstoffatom, an das sie gebunden sind, einen 5- oder 6-gliedrigen, gegebenenfalls substituierten Ring bedeuten; und anschliessend, sofern Y₁ Imidazol oder Halogen bedeutet, dieses durch Y austauscht, worin Y die für Formel l angegebenen Bedeutungen hat;
zu einer Verbindung der Formel V umsetzt.

15. Verfahren zur Herstellung einer Verbindung der Formel I 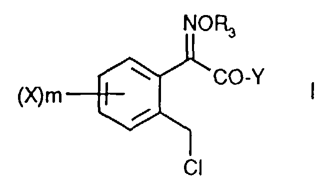 worin bedeuten:
X einen für die Reaktionen inerten Rest;
m 0 bis 4;
R₃ Wasserstoff, CH₃, CH₂F oder CHF₂;
Y eine Gruppe OR₄, N(R₅)₂, oder N(CH₃)OCH₃;
R₄ und R₅ unabhängig voneinander Wasserstoff oder C₁-C₈-Alkyl; oder
(R₅)₂ zusammen mit dem Stickstoffatom, an das sie gebunden sind, einen 5- oder 6-gliedrigen, gegebenenfalls substituierten Ring bedeuten;
dadurch gekennzeichnet, dass man eine Verbindung der Formel V 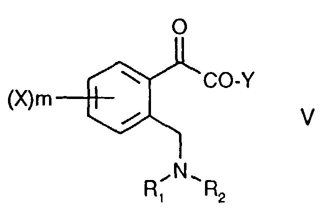 worin
X, m und Y die für Formel I angegebenen Bedeutungen haben und
R₁ und R₂ unabhängig voneinander C₁-C₆-Alkyl, C₁-C₆-Alkenyl, C₁-C₆-Alkoxyalkyl, oder C₃-C₆-Cycloalkyl oder
R₁ und R₂ zusammen mit dem Stickstoffatom einen gegebenenfalls substituierten 6- oder 7-gliedrigen Ring, der zusätzlich noch ein weiteres Stickstoffatom enthalten kann,
in beliebiger Reihenfolge
c1) mit O-Methylhydroxylamin oximiert; oder mit Hydroxylamin oximiert und anschliessend methyliert oder fluormethyliert oder difluormethyliert;
c2) mit einem Chlorameisensäureester umsetzt.

16. Eine Verbindung der Formel V 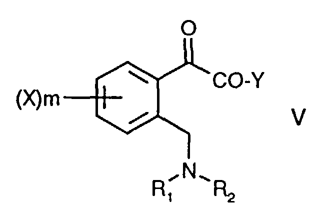 worin
X einen für die Reaktionen wie in Anspruch 1 beschrieben inerten Rest;
m 0 bis 4;
Y eine Gruppe OR₄, N(R₅)₂, oder N(CH₃)OCH₃;
R₄ und R₅ unabhängig voneinander Wasserstoff oder C₁-C₈-Alkyl; oder
(R₅)₂ zusammen mit dem Stickstoffatom, an das sie gebunden sind, einen 5- oder 6-gliedrigen, gegebenenfalls substituierten Ring bedeuten;
R₁ und R₂ unabhängig voneinander C₁-C₆-Alkyl, C₁-C₆-Alkenyl, C₁-C₆-Alkoxyalkyl, oder C₃-C₆- Cycloalkyl sind, oder
R₁ und R₂ zusammen mit dem Stickstoffatom einen gegebenenfalls substituierten 6- oder 7-gliedrigen Ring bilder, der zusätzlich zum Stickstoffatom noch ein weiteres Stickstoffatom enthalten kann.

17. Eine Verbindung gemäss Anspruch 16, worin
m 0;
Y eine Gruppe OR₄,;
R₄ C₁-C₈-Alkyl;
R₁ und R₂ unabhängig voneinander C₁-C₆-Alkyl, oder
R₁ und R₂ zusammen mit dem Stickstoffatom Piperidin bedeuten.

18. Die Verbindung gemäss Anspruch 17, worin
R₄ Ethyl;
R₁ und R₂ Methyl bedeuten.

19. Die Verbindung der Formel VII b

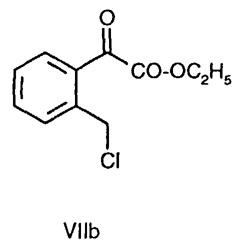

20. Die Verbindung der Formel Ib

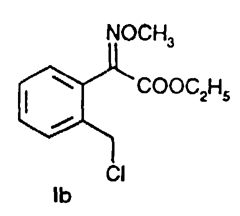

## Claims

1. A process for the preparation of a compound of formula I 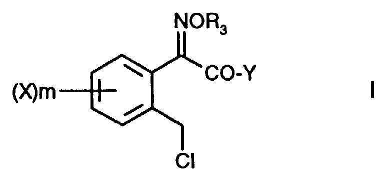 wherein:
X is a radical that is inert for the reactions;
m is from 0 to 4;
R₃ is hydrogen, CH₃, CH₂F or CHF₂;
Y is a group OR₄, N(R₅)₂ or N(CH₃)OCH₃;
R₄ and R₅ are each independently of the other hydrogen or C₁-C₈alkyl; or
(R₅)₂ together with the nitrogen atom to which they are bonded form a 5- or 6-membered, unsubstituted or substituted ring;
in which process
a) a compound of formula II 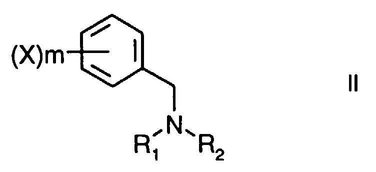 wherein
X and m are as defined for formula I, and
R₁ and R₂ are each independently of the other C₁-C₆alkyl, C₁-C₆alkenyl, C₁-C₆alkoxyalkyl or C₃-C₆cycloalkyl, or
R₁ and R₂ together with the nitrogen atom form an unsubstituted or substituted 6- or 7-membered ring that may contain in addition to the nitrogen atom a further nitrogen atom, is reacted, in an aprotic solvent, with an organolithium compound of formula III
Li-R₇ (III)
wherein R₇ is an organic anionic radical;
b) the resulting lithium complex is reacted with a compound of formula IV
Y₁-CO-CO-Y₁ IV
wherein
each of the substituents Y₁, which may be the same or different, is a group OR₄, N(R₆)₂ or N(CH₃)OCH₃ or imidazole or halogen;
R₄ is C₁-C₈alkyl;
R₆ is C₁-C₈alkyl; or
(R₆)₂ together with the nitrogen atom to which they are bonded form a 5- or 6-membered, unsubstituted or substituted ring;
and then, when Y₁ is imidazole or halogen, that group is replaced by Y, wherein Y is as defined for formula I;
to form a compound of formula V 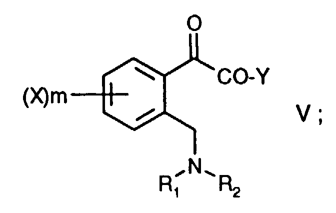
c) that compound is, in either order,
c1) oximated with O-methylhydroxylamine; or oximated with hydroxylamine and then methylated or fluoromethylated or difluoromethylated;
c2) reacted with a chloroformic acid ester.

2. A process according to claim 1, wherein reaction step a) is carried out at from 0°C to 120°C and reaction step b) is carried out at from -50°C to +30°C.

3. A process according to claim 1, wherein the solvent for reaction steps a) and b) is an ether or a hydrocarbon or a mixture thereof.

4. A process according to claim 3, wherein the hydrocarbon is hexane, benzene, toluene or xylene and the ether is tetrahydrofuran, diethyl ether, methyl tert-butyl ether, diisopropyl ether, dimethoxyethane, diethoxyethane or diethoxymethane.

5. A process according to claim 1, wherein the organolithium compound of formula III is butyllithium, sec-butyllithium, hexyllithium, lithium diisopropylamide (LDA), lithium hexamethyldisilazide or lithium tetramethylpiperidide (LTMP).

6. A process according to claim 5, wherein the organolithium compound of formula III is butyllithium.

7. A process according to claim 1, wherein in the compound of formula IV Y₁ is OR₃, especially OC₂H₅.

8. A process according to claim 1, wherein in the compound of formula II
m is 0, and
R₁ and R₂ are each independently of the other C₁-C₆alkyl, or
R₁ and R₂ together with the nitrogen atom form piperidine.

9. A process according to claim 1, wherein in reaction step a) there are used from 0.5 to 1.5 mol equivalents of the organolithium compound of formula III, based on the compound of formula II.

10. A process according to claim 1, wherein in reaction step b) there are used from 0.9 to 4 mol equivalents of the oxalic acid derivative of formula IV, based on the compound of formula II.

11. A process according to claim 1, wherein following reaction step b) the reaction mixture is adjusted to a pH of 7 or less.

12. A process according to claim 1, wherein chloroformic acid ethyl ester is used in reaction step c2).

13. A process according to claim 1, wherein in formulae I, II and V m is 0.

14. A process for the preparation of a compound of formula V 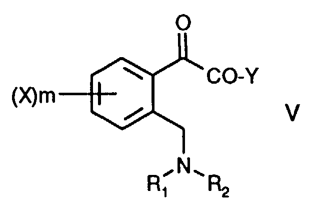 wherein:
X is a radical that is inert for the reactions;
m is from 0 to 4;
R₁ and R₂ are each independently of the other C₁-C₆alkyl, C₁-C₆alkenyl, C₁-C₆alkoxyalkyl or C₃-C₆cycloalkyl;
R₁ and R₂ together with the nitrogen atom form an unsubstituted or substituted 6- or 7-membered ring that may contain in addition to the nitrogen atom a further nitrogen atom, Y is a group OR₄, N(R₅)₂ or N(CH₃)OCH₃;
R₄ and R₅ are each independently of the other hydrogen or C₁-C₈alkyl; or
(R₅)₂ together with the nitrogen atom to which they are bonded form a 5- or 6-membered, unsubstituted or substituted ring;
in which process
a) a compound of formula II 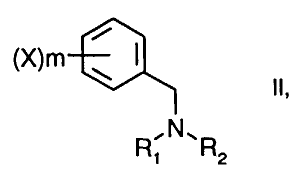 wherein X, m, R₁ and R₂ are as defined for formula V, is reacted, in an aprotic solvent, with an organolithium compound of formula III
Li-R₇ (III)
wherein R₇ is an organic anionic radical;
b) the resulting lithium complex is reacted with a compound of formula IV
Y₁-CO-CO-Y₁ IV
wherein
each of the substituents Y₁, which may be the same or different, is a group OR₄, N(R₆)₂ or N(CH₃)OCH₃ or imidazole or halogen;
R₄ is C₁-C₈alkyl;
R₆ is C₁-C₈alkyl; or
(R₆)₂ together with the nitrogen atom to which they are bonded form a 5- or 6-membered, unsubstituted or substituted ring;
and then, when Y₁ is imidazole or halogen, that group is replaced by Y, wherein Y is as defined for formula I;
to form a compound of formula V.

15. A process for the preparation of a compound of formula I 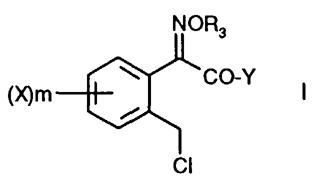 wherein:
X is a radical that is inert for the reactions;
m is from 0 to 4;
R₃ is hydrogen, CH₃, CH₂F or CHF₂;
Y is a group OR₄, N(R₅)₂ or N(CH₃)OCH₃;
R₄ and R₅ are each independently of the other hydrogen or C₁-C₈alkyl; or
(R₅)₂ together with the nitrogen atom to which they are bonded form a 5- or 6-membered, unsubstituted or substituted ring;
in which process a compound of formula V 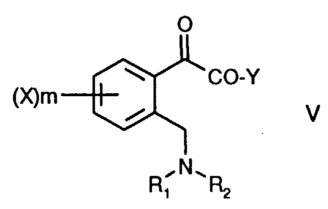 wherein
X, m and Y are as defined for formula I and
R₁ and R₂ are each independently of the other C₁-C₆alkyl, C₁-C₆alkenyl, C₁-C₆alkoxyalkyl or C₃-C₆cycloalkyl, or
R₁ and R₂ together with the nitrogen atom form an unsubstituted or substituted 6- or 7-membered ring that may additionally contain a further nitrogen atom,
is, in either order,
c1) oximated with O-methylhydroxylamine; or oximated with hydroxylamine and then methylated or fluoromethylated or difluoromethylated;
c2) reacted with a chloroformic acid ester.

16. A compound of formula V 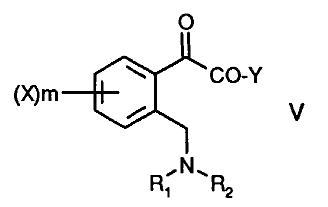 wherein
X is a radical that is inert for the reactions as described in claim 1;
m is from 0 to 4;
Y is a group OR₄, N(R₅)₂ or N(CH₃)OCH₃;
R₄ and R₅ are each independently of the other hydrogen or C₁-C₈alkyl; or
(R₅)₂ together with the nitrogen atom to which they are bonded form a 5- or 6-membered, unsubstituted or substituted ring;
R₁ and R₂ are each independently of the other C₁-C₆alkyl, C₁-C₆alkenyl, C₁-C₆alkoxyalkyl or C₃-C₆cycloalkyl, or
R₁ and R₂ together with the nitrogen atom form an unsubstituted or substituted 6- or 7-membered ring that may contain in addition to the nitrogen atom a further nitrogen atom.

17. A compound according to claim 16, wherein
m is 0;
Y is a group OR₄;
R₄ is C₁-C₈alkyl;
R₁ and R₂ are each independently of the other C₁-C₆alkyl, or
R₁ and R₂ together with the nitrogen atom form piperidine.

18. The compound according to claim 17 wherein
R₄ is ethyl;
R₁ and R₂ are methyl.

19. The compound of formula VIIb

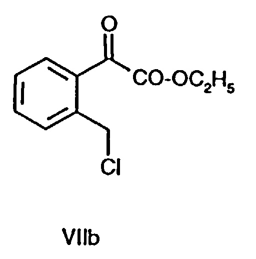

20. The compound of formula Ib

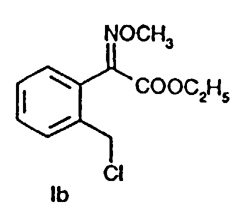

## Revendications

1. Procédé de préparation d'un composé de formule I 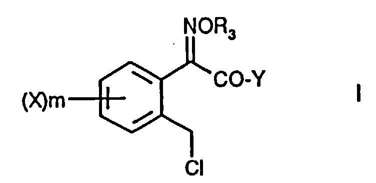 dans laquelle :
X est un groupement inerte pour les réactions ;
m est compris entre 0 et 4 ;
R₃ est un atome d'hydrogène, CH₃, CH₂F ou CHF₂;
Y est un groupe OR₄, N(R₅)₂ ou N(CH₃)OCH₃;
R₄ et R₅ sont indépendamment l'un de l'autre un atome d'hydrogène ou un alkyle en C₁ à C₈ ; ou
(R₅)₂, avec l'atome d'azote auquel ils sont liés, forment un cycle à 5 ou 6 membres, éventuellement substitué ;
caractérisé en ce que l'on fait réagir
a) un composé de formule II 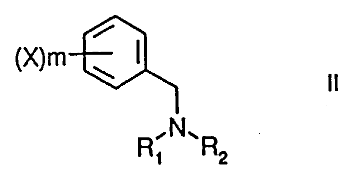 dans laquelle
X et m ont la signification indiquée pour la formule I et
R₁ et R₂ sont indépendamment l'un de l'autre un alkyle en C₁ à C₆, un alcényle en C₁ à C₆, un alcoxyalkyle en C₁ à C₆ ou un cycloalkyle en C₃ à C₆, ou
R₁ et R₂, avec l'atome d'azote, forment un cycle à 6 ou 7 membres, éventuellement substitué, qui, outre l'atome d'azote, peut comporter un autre atome d'azote, avec un composé organique de lithium de formule III
Li-R₇ (III)
dans laquelle R₇ représente un groupement anionique organique, dans un solvant aprotique ;
b) le complexe de Li ainsi obtenu avec un composé de formule IV
Y₁-CO-CO-Y₁ IV
dans laquelle
Y₁ est identique ou différent et représente un groupe OR₄, N(R₆)₂, N(CH₃)OCH₃, un imidazole ou un halogène ;
R₄ est un alkyle en C₁ à C₈;
R₆ est un alkyle en C₁ à C₈; ou
(R₆)₂, avec l'atome d'azote auquel ils sont liés, représentent un cycle à 5 ou 6 membres, éventuellement substitué; puis, dans la mesure où Y₁ représente un imidazole ou un halogène, on échange celui-ci par Y, où Y a la signification indiquée pour la formule I ;
en un composé de formule V ; 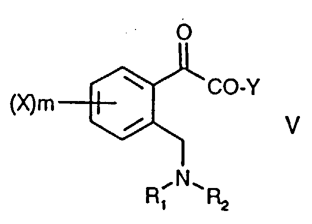
c) et, consécutivement et selon une séquence quelconque
c1) on oxime celui-ci avec une O-méthylhydroxylamine; ou on l'oxime avec une hydroxylamine et on le méthylise ou on le fluorométhylise ou on le difluorométhylise ;
c2) on fait réagir celui-ci avec un ester de l'acide chloraminoferrique.

2. Procédé selon la revendication 1, dans lequel l'étape de réaction a) est réalisée à une température comprise entre 0 °C et 120 °C et l'étape de réaction b) est réalisée à une température comprise entre - 50 et + 30 °C.

3. Procédé selon la revendication 1, dans lequel le solvant pour les étapes de réaction a) et b) est un éther ou un composé hydrocarboné ou un mélange de ceux-ci.

4. Procédé selon la revendication 3, dans lequel le composé hydrocarboné est l'hexane, le benzène, le toluène, le xylène, et dans lequel l'éther est le tétrahydrofurane, l'éther diéthylique, le méthyl-tert. butyléther, l'éther diisopropylique, le diméthoxyéthane, le diéthoxyéthane ou le diéthoxyméthane.

5. Procédé selon la revendication 1, dans lequel le composé organique de lithium de formule III est le butyllithium, le sec. butyllithium, l'hexyllithium, le diisopropylamide de lithium (LDA), l'hexaméthyldisilazide de lithium ou le tétraméthylpipéridide de lithium (LTMP).

6. Procédé selon la revendication 5, dans lequel le composé organique de lithium de formule III est le butyllithium.

7. Procédé selon la revendication 1, dans lequel, dans le composé de formule IV, Y₁ représente OR₃ ; en particulier OC₂H₅.

8. Procédé selon la revendication 1, dans lequel, dans le composé de formule II
m vaut 0 et
R₁ et R₂ sont indépendamment l'un de l'autre un alkyle en C₁ à C₆ ou
R₁ et R₂ forment avec l'atome d'azote la pipéridine.

9. Procédé selon la revendication 1, dans lequel on utilise à l'étape de réaction a) 0,5 à 1,5 équivalent molaire du composé organique de lithium de formule III, sur la base du composé de formule II.

10. Procédé selon la revendication 1, dans lequel on utilise à l'étape de réaction b) 0,9 à 4 équivalents molaires du dérivé d'acide oxalique de formule IV, sur la base du composé de formule II.

11. Procédé selon la revendication 1, caractérisé en ce que le mélange réactionnel est ajusté après l'étape de réaction b) à un pH inférieur ou égal à 7.

12. Procédé selon la revendication 1, dans lequel on utilise à l'étape de réaction c2) l'éthylester de l'acide chloraminoferrique.

13. Procédé selon la revendication 1, dans lequel, dans les formules I, II et V, m = 0.

14. Procédé de préparation d'un composé de formule V 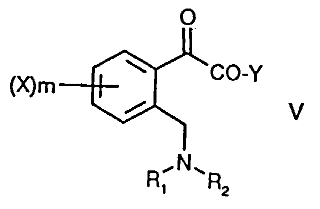 dans laquelle
X est un groupement inerte pour les réactions ;
m est compris entre 0 et 4 ;
R₁ et R₂ sont indépendamment l'un de l'autre un alkyle en C₁ à C₆, un alcényle en C₁ à C₆, un alcoxyalkyle en C₁ à C₆ ou un cycloalkyle en C₃ à C₆,
R₁ et R₂, avec l'atome d'azote, forment un cycle à 6 ou 7 membres, éventuellement substitué, qui, outre l'atome d'azote, peut comporter un autre atome d'azote,
Y est un groupe OR₄, N(R₅)₂ ou N(CH₃)OCH₃;
R₄ et R₅ sont indépendamment l'un de l'autre un atome d'hydrogène ou un alkyle en C₁ à C₈ ; ou
(R₅)₂, avec l'atome d'azote auquel ils sont liés, forment un cycle à 5 ou 6 membres, éventuellement substitué ;
caractérisé en ce que l'on fait réagir
a) un composé de formule II 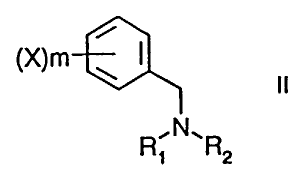 dans laquelle X, m, R₁ et R₂ ont les significations indiquées pour la formule V ; avec un composé organique de lithium de formule III
Li-R₇ (III)
dans laquelle R₇ représente un groupement anionique organique, dans un solvant aprotique ;
b) le complexe de Li ainsi obtenu avec un composé de formule IV
Y₁-CO-CO-Y₁ IV
dans laquelle
Y₁ est identique ou différent et représente un groupe OR4, N(R₆)₂, N(CH₃)OCH₃, un imidazole ou un halogène ;
R₄ est un alkyle en C₁ à C₈ ;
R₆ est un alkyle en C₁ à C₈ ; ou
(R₆)₂, avec l'atome d'azote auquel ils sont liés, représentent un cycle à 5 ou 6 membres, éventuellement substitué; puis, dans la mesure où Y₁ représente un imidazole ou un halogène, on échange celui-ci par Y, où Y a la signification indiquée pour la formule I ;
pour donner un composé de formule V.

15. Procédé de préparation d'un composé de formule I 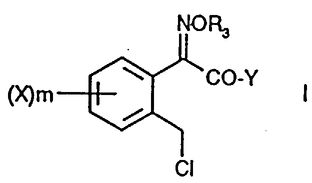 dans laquelle :
X est un groupement inerte pour les réactions ;
m est compris entre 0 et 4 ;
R₃ est un atome d'hydrogène, CH₃, CH₂F ou CHF₂ ;
Y est un groupe OR₄, N(R₅)₂ ou N(CH₃)OCH₃;
R₄ et R₅ sont indépendamment l'un de l'autre un atome d'hydrogène ou un alkyle en C₁ à C₈ ; ou
(R₅)₂, avec l'atome d'azote auquel ils sont liés, forment un cycle à 5 ou 6 membres, éventuellement substitué ;
caractérisé en ce qu'un composé de formule V 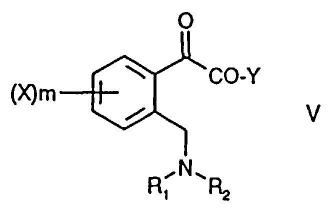 dans laquelle
X, m et Y ont les significations indiquées pour la formule I et R₁ et R₂ sont indépendamment l'un de l'autre un alkyle en Ci à C₆, un alcényle en C₁ à C₆, un alcoxyalkyle en C₁ à C₆ ou un cycloalkyle en C₃ à C₆, ou
R₁ et R₂, avec l'atome d'azote, forment un cycle à 6 ou 7 membres, éventuellement substitué, qui, peut en outre comporter un autre atome d'azote,
selon une séquence quelconque,
c1) est oximé avec une O-méthylhydroxylamine; ou est oximé avec une hydroxylamine puis est méthylisé ou fluorométhylisé ou difluorométhylisé ;
c2) est mis à réagir avec un ester de l'acide chloraminoferrique.

16. Composé de formule V 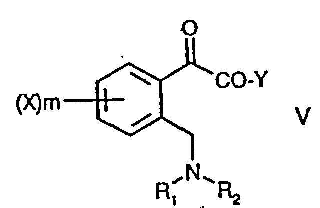 dans laquelle :
X est un groupement inerte pour les réactions tel que décrit dans la revendication 1 ;
m est compris entre 0 et 4 ;
Y est un groupe OR₄, N(R₅)₂ ou N(CH₃)OCH₃;
R₄ et R₅ sont indépendamment l'un de l'autre un atome d'hydrogène ou un alkyle en C₁ à C₈ ; ou
(R₅)₂, avec l'atome d'azote auquel ils sont liés, forment un cycle à 5 ou 6 membres, éventuellement substitué ;
R₁ et R₂ sont indépendamment l'un de l'autre un alkyle en C₁ à C₆, un alcényle en C₁ à C₆, un alcoxyalkyle en C₁ à C₆ ou un cycloalkyle en C₃ à C₆ ; ou
R₁ et R₂, avec l'atome d'azote, forment un cycle à 6 ou 7 membres, éventuellement substitué, qui, outre l'atome d'azote, peut comporter un autre atome d'azote.

17. Composé selon la revendication 16, dans lequel
m vaut 0 ;
Y est un groupe OR₄;
R₄ est un alkyle en C₁ à C₈;
R₁ et R₂ sont indépendamment l'un de l'autre un alkyle en C₁ à C₆ ; ou
R₁ et R₂, avec l'atome d'azote, représentent une pipéridine.

18. Composé selon la revendication 17, dans lequel
R₄ est l'éthyle ;
R₁ et R₂ représentent le méthyle.

19. Composé de formule VIIb

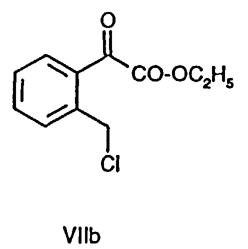

20. Composé de formule Ib
